# EUROPEAN PATENT APPLICATION

(11) **EP 3 407 084 A1**
(43) Date of publication of application: **28.11.2018**
(21) Application number: 16885713.4
(22) Date of filing: 22.01.2016
(51) Int. Cl.: G01S 7/526

(54) **ULTRASOUND IMAGING DEVICE AND METHOD**

(71) Applicant: Shenzhen Genorivision Technology Co., Ltd., Shenzhen, Guangdong 518054 (CN)
(72) Inventor: CAO, Peiyan, Shenzhen Guangdong 518054 (CN); ZHANG, Lin, Shenzhen Guangdong 518054 (CN); ZHANG, Jia, Shenzhen Guangdong 518054 (CN)
(74) Representative: Beck Greener
(86) International application number: PCT/CN2016/071766
(87) International publication number: WO 2017/124440

(57) **Abstract**

An ultrasound imaging device, the device comprising a first chip (202) and a second chip (206), the first chip (202) receiving an ultrasound signal and generating a digital signal representative of the ultrasound signal, the ultrasound signal being an analog signal, the second chip (206) processing the digital signal from the first chip(202) for ultrasound imaging. The ultrasound imaging device has reduced power consumption. Further disclosed is a corresponding ultrasound imaging method.

## Description

### TECHNICAL FIELD

The disclosure belongs to the field of digital imaging, and more particularly to a device and method for ultrasonic imaging.

### BACKGROUND

Mechanical vibration wave with frequencies above 20,000 Hz is called ultrasonic wave, or ultrasound. Ultrasound imaging, usually used as an examination method for disease diagnosis, for example, makes use of the physical characteristics of ultrasound and the differences in the acoustic properties of human organs, to display and record the differences in the form of waveform, curve or image for disease diagnosis. The medical high frequency ultrasonic wave is produced by the piezoelectric transducer on the ultrasonic diagnostic instrument, which is also called the probe and can convert the electric energy into the ultrasonic energy and transmit the ultrasonic wave. At the same time, the probe can also accept the returned ultrasonic wave and convert it into electrical signal. The electrical signal is processed and used for ultrasonic imaging. The ultrasonic diagnostic equipment is not as expensive as CT or MRI equipment, can obtain the image of any section of the organ, and can also observe the movement of the motion organs, with advantages of rapid imaging, timely diagnosis, no pain, no danger, and the non-injury examination. Therefore, this equipment has been widely used in clinical practice, and is an important part of medical imaging.

Existing ultrasonic imaging equipment has some limitations restricting the broadening of the application scope of ultrasound imaging. For example, the volume and weight of the ultrasonic imaging equipment are relatively large, and the power consumption is high, increasing the difficulty in utilizing portable ultrasonic equipment.

### SUMMARY

In view of the above-described problems, it is an objective of the invention to provide a device and method for ultrasonic imaging.

To achieve the above objective, according to one embodiment of the invention, there is provided a device for ultrasonic imaging, comprising: a first chip configured to receive an ultrasonic signal, the ultrasonic signal being an analog signal, and to generate a digital signal corresponding to the ultrasonic signal; and a second chip configured to process the digital signal transmitted from the first chip for ultrasonic imaging.

In a class of this embodiment, the first chip is stacked on the second chip to form a three-dimensional stacked chip package.

In a class of this embodiment, the second chip comprises a digital signal processor prepared by an application-specific integrated circuit technology.

In a class of this embodiment, the first chip comprises a plurality of signal channels, each of which comprises a low noise amplifier configured to generate a first amplification signal by amplifying the ultrasonic signal; a variable gain amplifier configured to generate a second amplification signal by amplifying the first amplification signal; and an analog-to-digital converter configured to convert the second amplification signal into the digital signal.

In a class of this embodiment, the low noise amplifier comprises a differential current reuse low noise amplifying circuit; the differential current reuse low noise amplifying circuit comprises: a first branch circuit comprising a first P type metal oxide semiconductor transistor and a first N type metal oxide semiconductor transistor which are connected in series, and a second branch circuit comprising a second P type metal oxide semiconductor transistor and a second N type metal oxide semiconductor transistor which are connected in series; gates of the first and second P type metal oxide semiconductor transistors and the first and second N type metal oxide semiconductor transistors are coupled with an input capacitor to receive a differential input signal; drains of the first and second P type metal oxide semiconductor transistors and the first and second N type metal oxide semiconductor transistors output the first amplification signal.

In a class of this embodiment, the analog-to-digital converter comprises: a first stage converter configured to generate a converted first digital signal and an amplified first residual analog signal according to the second amplification signal; a second stage analog-to-digital converter based on a voltage controlled oscillator, which is configured to generate a second digital signal according to the first residual analog signal; and a digital calibration circuit configured to generate a calibrated digital signal according to the first digital signal and the second digital signal.

In a class of this embodiment, the first stage converter comprises: a flash analog-to-digital converter configured to generate the first digital signal according to the second amplification signal and a first reference signal; a digital to analog converter configured to generate a first analog signal according to the first digital signal and the first reference signal; an addition and subtraction device configured to generate a residual signal according to the second amplification signal and the first analog signal; and a residual amplifier configured to generate a first residual analog signal according to the residual signal.

In a class of this embodiment, the residual amplifier comprises a voltage multiplier circuit based on a charge pump; the voltage multiplier circuit is configured to: store paired residual signals in a first stage, and combine and output the paired residual signals in a second stage to yield the first residual analog signal.

In a class of this embodiment, the second stage analog-to-digital converter comprises: a sample-and-hold circuit configured to sample and maintain the first residual analog signal to generate a sampling voltage signal; a voltage to current conversion circuit configured to convert the sampling voltage signal to a sampling current signal; a current-controlled oscillator configured to generate an oscillating signal based on the sampling current signal; a bidirectional counter configured to count according to the oscillating signal; and an addition and subtraction device configured to calculate the second digital signal according to a counting result from the bidirectional counter.

In accordance with another embodiment of the invention, provided is a portable ultrasonic testing equipment, comprising: an ultrasonic transducer configured to generate an ultrasonic signal by detection; the aforesaid device for ultrasonic imaging, which is configured to generate a digital signal for ultrasonic imaging according to the ultrasonic signal; and a display unit configured to image according to the digital signal.

In accordance with still another embodiment of the invention, provided is a method of ultrasonic imaging, comprising: receiving, by a first chip, an ultrasonic signal, the ultrasonic signal being an analog signal; generating, by the first chip, a digital signal corresponding to the ultrasonic signal; and processing, by a second chip, the digital signal transmitted from the first chip for ultrasonic imaging.

In another aspect, the disclosure provides an imaging method of portable ultrasonic testing equipment, the method comprising: generating an ultrasonic signal according to ultrasonic energy exchange; generating, by the aforesaid device for ultrasonic imaging, a digital signal for ultrasonic imaging according to the ultrasonic signal; and imaging according to the digital signal.

Further provided is a method of producing ultrasonic imaging device, the method comprising providing a component unit of the aforesaid device for ultrasonic imaging.

It should be understood that this disclosure does not aim at identifying the key or important features of the embodiments of the disclosure, nor is it intended to restrict the scope of the disclosure. Other features of this disclosure will be easier to understand in combination with the following description.

### BRIEF DESCRIPTION OF THE DRAWINGS

Advantages, features and objectives of this disclosure will become more apparent through a more detailed description of some of the embodiments with the attached drawing.
FIG. 1 illustrates a simplified block diagram of an environment in which some embodiments of the disclosure can be implemented;
FIG. 2 shows a stereogram of a chip stacking for an ultrasonic imaging device according to one embodiment of the disclosure;
FIG. 3 shows a block diagram of a device for ultrasonic imaging according to one embodiment of the disclosure;
FIG. 4 shows a circuit diagram of a low noise amplifier in FIG. 3;
FIG. 5 shows a block diagram of an analog-to-digital converter in FIG. 3;
FIG. 6 shows a specific block diagram of the analog-to-digital converter in FIG. 5;
FIG. 7 shows a circuit diagram of a residual amplifier shown in FIG. 6;
FIG. 8 is a block diagram of a second stage analog-to-digital converter in FIG. 6; and
FIG. 9 shows a flow chart of certain methods according to some embodiments of the disclosure.

### DETAILED DESCRIPTION OF THE EMBODIMENTS

To further illustrate the invention, experiments detailing a device and method for ultrasonic imaging are described below. It should be noted that the following examples are intended to describe and not to limit the invention.

As described in this disclosure, the term "comprise" and its variants can be understood as open terms, which means "including but not limited to". The term "based on" can be understood as "at least partially based on". The term "one embodiment" can be understood as "at least one embodiment". The term "another embodiment" can be understood as "at least another embodiment". The term "logic" refers to modules such as circuit devices used for timing functions. In addition, in the disclosure, the term "signal flow" and "data stream" can be exchanged for purposes of convenience of discussion. Other obvious and suggestive definitions are included below.

The ultrasonic signal acquisition and processing device in the existing ultrasonic imaging equipment is an important part of the ultrasonic imaging system. The ultrasonic signal acquisition and processing device includes a plurality of signal receiving channels, and the ultrasonic signals are output to a display processor through the transducer, the low noise amplifier (LNA), the variable gain amplifier (VGA), the analog-to-digital converter (ADC), the digital beamformer, the digital signal processor (DSP) and so on in each receiving channel for imaging. Typical ultrasonic imaging systems usually contain 32 to 256 signal receiving channels. Due to the large quantity of receiving channels, a large number of unit circuits (such as low noise amplifier, variable gain amplifier, analog-to-digital converter, etc.) are needed to build such an ultrasonic system with discrete components, which makes the entire ultrasonic device large and expensive, and leads to considerable power consumption.

In addition, the ultrasonic signal acquisition and processing chip in the existing ultrasonic imaging equipment usually includes at least three chips (i.e., gain amplification chips, analog digital conversion chips, and digital signal processing chips). The gain amplification chip and analog digital conversion chip are all versatile general chips, and the digital signal processing chips are usually implemented using a field programmable gate array (FPGA). FPGA has the advantages of good generality and easy development. In addition, the above three chips in the existing ultrasonic imaging device are usually arranged on the same plane in the same substrate, and are connected by electric wirings. The configuration is flexible, but has large power consumption.

For example, in an existing 64 channel ultrasonic detector, the power consumed by each channel of the analog front end is 75 milliwatt (mW), of which the LNA and VCA consume 20 mW, and the ADC consumes 55 mW. For the 64 channels, the total analog front end consumes 4.8 W of power. The FPGA power consumption of Xilinx in the digital signal processing module is 1.5 W. The whole scheme consumes at least 6.3 W of power. This power consumption makes the scheme only suitable for the base station system with grid power supply, but not suitable for portable ultrasonic detectors.

To solve the above mentioned and other potential problems, some embodiments of this disclosure provide devices and methods for ultrasonic imaging. In embodiments of the disclosure, for example, after the device for ultrasonic imaging receives an ultrasonic analog signal sent by an ultrasonic transducer, the analog front end chip in the device amplifies and performs the analog-digital conversion on the ultrasonic analog signal to obtain a digital signal for ultrasonic imaging. The digital signal is then transmitted to the digital signal processing chip for digital signal processing. Thus, image data suitable for display devices such as liquid crystal displays are obtained. By dividing the chip function, integrating the divided functions into two different chips, and redesigning the circuit structure, the device of the disclosure can greatly reduce the power consumption of the ultrasonic equipment, and the device for ultrasonic imaging according to certain embodiments of the disclosure can be applied to the portable ultrasonic imaging device.

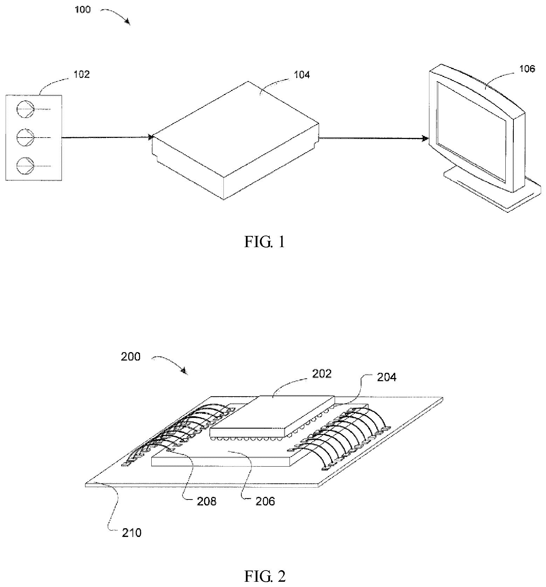

FIG. 1 illustrates a simplified block diagram of an environment in which some embodiments of the disclosure can be implemented. The environment 100 comprises an ultrasonic transducer 102, a host 104 and a display 106. The host 104 comprises a device for ultrasound imaging and other peripherals. The ultrasonic transducer 102, for example, can be used for transmitting ultrasonic waves to scan the human body, receiving echo signals and converting them into analog electrical signals corresponding to the ultrasonic signals. The analog electrical signals are transmitted to the ultrasonic imaging device of the host 104. The device for ultrasonic imaging then processes the analog electrical signals, such as amplifying, filtering, digital analog conversion, digital signal processing (DSP), etc. to obtain image data suitable for display on the display 106. The display 106, for example, is a liquid crystal display (LCD), which can display images such as human tissue after receiving image data.

Although the ultrasonic transducer 102, the host 104 and the display 106 are separated in FIG. 1, it is understood that these components can be integrated into one instrument. The configuration shown in FIG. 1 is only an example, rather than a limitation on the scope of the disclosure. The three components can be selectively separated and assembled without departing from the scope of the disclosure.

FIG. 2 shows a stereogram of a chip stacking 200 for an ultrasonic imaging device according to one embodiment of the disclosure. The chip stacking 200 can be included, for example, in the host 102 shown in FIG. 1, for processing an analog ultrasonic signal from the ultrasonic transducer 102 such as amplification, conversion and so on. In this example, the chip stacking 200 is implemented as a three-dimensional (3D) chip stack, including a first chip (analog front end chip) 202, a second chip (digital signal processing chip) 206, and a package substrate 210. The package substrate 210 is used to support the second chip 206. A plurality of electrical connection terminals is disposed on the package substrate 210 (in Fig.2, two row terminals are shown, and other arrangements can be used). Also, a plurality of electrical connection terminals 208 is disposed on the second chip 206 for transmitting signals between the second chip 206 and peripheral circuit devices or displays. The first chip 202 is in vertical integration with the second chip 206 through multiple Copper Pillar Micro Bump or similar technologies. The high flux and high speed data between the two chips are transmitted by Micro Bump, thus reducing the channel capacitance and power consumption. The digital signal processing chip is electrically connected to the package substrate 210 through the wire bonding. The chip stacking 200 is subsequently encapsulated in a single device for use by ultrasound imaging equipment.

In the above embodiments, the three-dimensional encapsulation technology can greatly reduce the parasitic capacitance of the signal transmission channels between two chips, thus effectively reducing the power consumption for transmitting a large number of high-speed data between the two chips. In addition, the use of three-dimensional packaging technology can greatly reduce the package area of the overall solution, thus minimizing the end product, such as reducing the weight and volume of the portable ultrasonic detector. In implementation, the Chip on Wafer on Substrate (CoWoS) encapsulation technology on the substrate and F2F FlipStack CSP encapsulation technology can be used to integrate multiple chips vertically to reduce interconnect capacitance and encapsulation area. Although FIG. 2 shows a three-dimensional stacking mode of the package substrate 210- second chip 206- first chip 202, other three dimensional stacking modes can be used. Compared with the above existing ultrasonic imaging devices, the power consumption of the ultrasonic equipment can be greatly reduced by the redesign of the circuit, the chip customization integration and the three-dimensional stacking according to the embodiment of the disclosure.

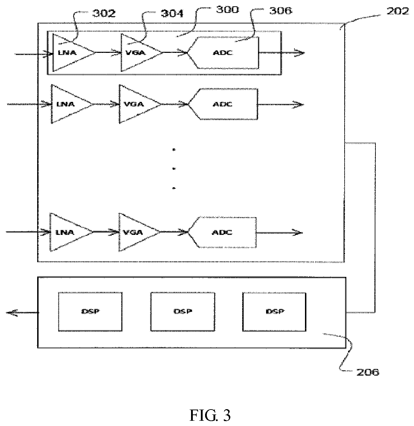

Referring to FIG. 3, FIG. 3 shows a block diagram of a device for ultrasonic imaging according to one embodiment of the disclosure. The device can be part of the host 104 in FIG. 1 and corresponds to the device shown in FIG. 2. The device comprises a first chip 202 and a second chip 206. The first chip 202 comprises a plurality of channels 300, and each channel 300 comprises a low noise amplifier (LNA) 302, a variable gain amplifier (VGA) 304 and an analog-to-digital converter (ADC) 306. The low noise amplifier 302 receives a weak ultrasonic pulse feedback signal from an ultrasonic transducer and amplifies and transfers the signal to the variable gain amplifier 304. The variable gain amplifier 304 is, for example, a programmable variable gain amplifier (PGA) or a voltage controlled variable gain amplifier (VCA). After being further amplified by the variable gain amplifier 304, the analog signal is transmitted to the analog-to-digital converter 306 and is converted to a digital signal via the analog-to-digital converter. The digital signal is further processed by the second chip 206. The digital signal processing chip is composed of a large number of DSP modules, and is configured to filter, balance and denoise the ultrasonic pulse feedback signal which is amplified and digitized by the analog front end, and to send the processing results to the operating system (OS) and the user interface (UI) for the user to analyze and judge.

The intensity of the ultrasonic pulse feedback signal received by the ultrasonic transducer is generally weak to only milli V (mV) magnitude. If the signal is amplified to an ideal input voltage level for subsequent ADCs (about 100mV magnitude), the amplification circuit needs to provide at least 40 dB gain. In one embodiment, a low noise amplification circuit can be arranged at the input end of the analog front end chip so as to ensure that the amplifier circuit does not introduce too much circuit noise in the process of amplifying the desired signal. In this way, the adverse effect against the signal to noise ratio of the input end of the analog-to-digital converter can be avoided. On the one hand, the low noise amplifier should provide sufficient gain for the input useful signal to suppress the noise of the subsequent circuit; on the other hand, to ensure as little noise as possible is introduced. To achieve the above two design requirements, the input devices of the LNA provide larger transconductance (GM), which may lead to larger power consumption. In the conventional differential input low noise amplifier design, single ended current design is usually used. In the single ended current design, only one input device, such as N type metal oxide semiconductor transistor (NMOS), provides transconductance.

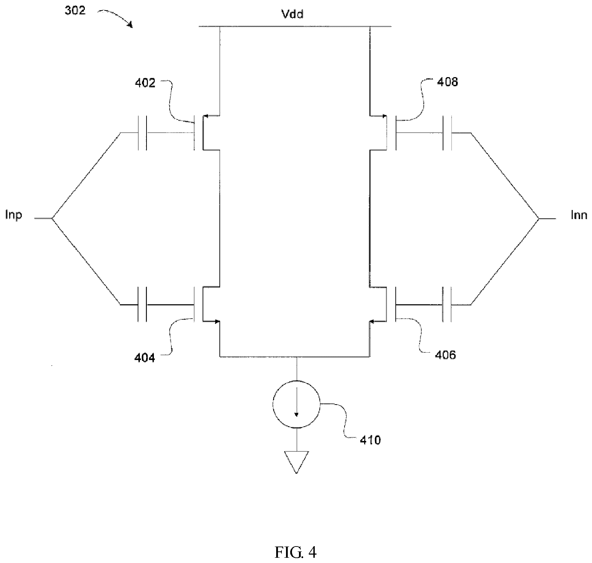

Referring to FIG. 4, FIG. 4 shows a circuit diagram of the LNA in FIG. 3. As shown in FIG. 4, the current reuse differential input low noise amplifier 302 comprises two branches, and each branch is disposed between the Vdd and the current source 410. The first branch comprises a P type metal oxide semiconductor transistor (PMOS) 402 and an N type metal oxide semiconductor transistor (NMOS) 404 which are connected in series. The second branch comprises PMOS 408 and NMOS 406 which are connected in series. The gates of the PMOS and the NMOS are coupled to the input capacitors to receive differential input signals, and the drains of the PMOS and the NMOS output the first amplification signals of. In the example shown in FIG. 4, the two input devices (NMOS and PMOS) are provided with transconductance by the single ended current, which significantly reduces the power consumption of the LNA.

Through current reuse, the circuit achieves two times the transconductance of conventional circuits in the case of consuming the same current. In other words, the low noise amplifier with current reuse can reduce the power consumption by half of the circuit on the basis of the same noise performance as the conventional low noise amplifier. As the ultrasonic detector usually requires a large number of signal channels (such as 64 signal channels), each channel needs a low noise amplification circuit, so reducing half of the power consumption of the low noise amplifier circuit will significantly reduce the power consumption of the whole ultrasonic detector.

As mentioned above, the implementation scheme of existing ultrasonic detectors adopts a pipeline analog-to-digital converter (Pipeline ADC) to complete the conversion of analog signals to digital signals. Therefore, the analog-to-digital converter in the existing ultrasonic imaging equipment consumes a lot of power when performing analog-to-digital conversion, for example, the ADC of each channel consumes 50 mW. For the 64 channel devices, this will consume a total of 3.2 W. The power consumption is absolutely unacceptable for battery powered portable ultrasound imaging equipment. In addition, the signal sampling frequency of the ultrasonic detector is usually between 20 mHz and 60 mHz, and the precision of analog-to-digital conversion requires to be 12 bits or even 14 bits. Such high sampling frequency determines the integral type analog-to-digital converter (Integrating ADC), the successive approximation analog-to-digital converter (SAR ADC) and the integral differential mode analog-to-digital converter (Sigma-Delta ADC) cannot meet the speed requirements of the analog digital conversion of the ultrasonic detector. In addition, although the Flash ADC can meet the requirement of frequency, the design of more than 8 bits or more cannot be achieved.

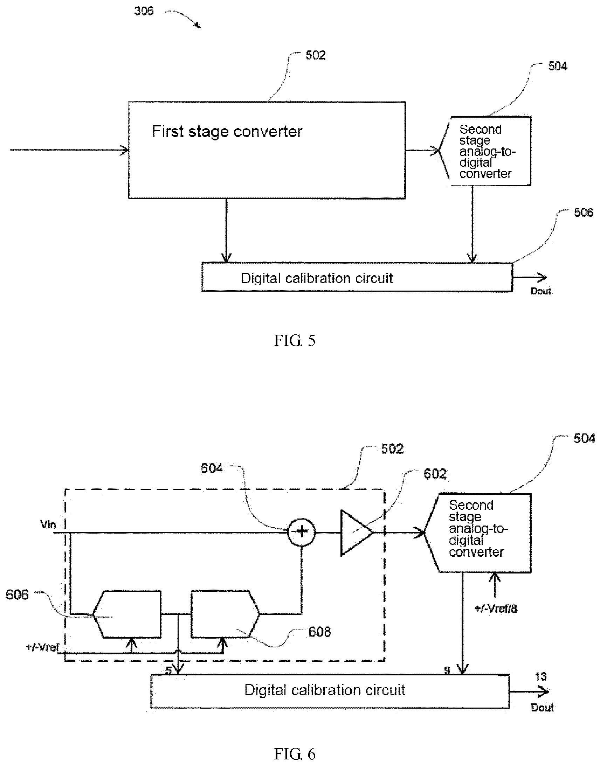

To solve the above problems, one embodiment of the disclosure provides a novel ADC to meet the needs of portable ultrasonic imaging equipment. Referring to FIG. 5, FIG. 5 shows a block diagram of the ADC in FIG. 3. The analog-to-digital converter 306 in FIG. 5 is a hybrid ADC working in the voltage domain and the time domain, which comprises a first stage converter 502, a second stage analog-to-digital converter 504 based on a voltage controlled oscillator, and a digital calibration circuit 506. The first stage converter 502 operates in a voltage domain and can be operable to generate a converted first digital signal and an amplified first residual analog signal based on a second amplification signal from the variable gain amplifier 304, in which the first digital signal is a number of bits of higher position in the digital bit representing the ultrasonic signal, such as the previous 5 bits. The second stage analog-to-digital converter 504, for example, is an analog-to-digital converter based on a voltage controlled oscillator and working in the time domain, which is operable to generate the second digital signal according to the first residual analog signal. The second digital signal is lower bits in the digital bit representing the ultrasonic signal, such as the later 9 bits. It can be understood that the digital bits (5 bits and 9 bits) are only used for examples, rather than for restrictions, and other digital bits can be used. The digital calibration circuit 506 is operable to generate a calibrated digital signal Dₒᵤₜ according to the first digital signal and the second digital signal.

By using the configuration structure of the dual stage converter shown in FIG. 5, the first stage converter and the second stage analog-to-digital converter can be configured flexibly. For example, a second stage analog-to-digital converter can use an analog-to-digital converter based on a voltage controlled oscillator (VCO). The analog-to-digital converter based on a voltage controlled oscillator can realize the high dynamic range analog to digital conversion of small signals. Analog-to-digital converters based on voltage controlled oscillators consume less power than conventional voltage mode ADCs. This advantage is more significant in small size production processes (such as 65 nm production process).

In addition, the voltage controlled oscillator requires a much smaller input voltage level than the conventional voltage mode analog-to-digital converter, so that the residue of the first stage analog-to-digital converter can be converted directly by a voltage-controlled oscillator without a large gain. This makes it possible to reduce the requirements of the first stage converter, such as the use of a less power operational amplifier (Opamp). In addition, because the VCO is a small input signal, no residue is needed between the two stage converters. As long as the counter of the voltage controlled oscillator counting counter is guaranteed to have enough counting range, the error of the first stage can be calibrated and corrected. This can further reduce the requirements for the first stage converter.

Referring to FIG. 6, FIG. 6 shows a more specific block diagram of the analog-to-digital converter in FIG. 5. The ADC in FIG. 6 comprises the first stage converter (shown in dotted box) 502, a second stage analog-to-digital converter 504 and a digital calibration circuit 506. Similar to the block diagram in FIG. 5, the first stage converter 502 outputs the first 5 digital bits to the digital calibration circuit 506, and the second analog-to-digital converter outputs the later 9 digital bits to the digital calibration circuit 506. The digital calibration circuit 506 calibrates and combines the digital bits of the two parts, and outputs a bit combination Dₒᵤₜ with a specific digit (for example, 13 bits), and the excess one bit is used for the residue of digital calibration.

In FIG. 6, the first stage converter 502 comprises a flash analog-to-digital converter 606, a digital to analog converter 608, an addition and subtraction device 604 and a residual amplifier 602. The flash analog-to-digital converter 606 can be operated to generate a first digital signal based on a second amplification signal Vin and a first reference signal V_{ref}, such as the first 5 digital bits described above. It is known that the flash analog-to-digital converter is not suitable for converting bits greater than 8. However, the device has the advantages of high speed and simple circuit structure, so it can act as the first stage conversion. This is because, in the two stage conversion of the embodiments, the first stage conversion usually requires only a few previous bits (for example, 5 bits).

The DAC 608 is operable to generate the first analog signal according to the first digital signal and the first reference signal Vref. The addition and subtraction device 604 then calculates the residual signal used to generate the later 9 digit bits according to the second amplification signal Vin and the first analog signal. The residual signal is then amplified by the residual amplifier 602 to generate the first residual analog signal. The second stage analog-to-digital converter 504, for example, is a second stage analog-to-digital converter based on a voltage controlled oscillator, which has a low requirement for the residual signal and input voltage, as described above. For example, see FIG. 6, the second stage analog-to-digital converter 504 generates the later 9 digital bits according to the first residual analog signal and the second reference signal Vref/8. As mentioned above, the power consumption can be significantly reduced because the second stage analog-to-digital converter uses a scheme based on a voltage controlled oscillator and has low requirements for the input voltage.

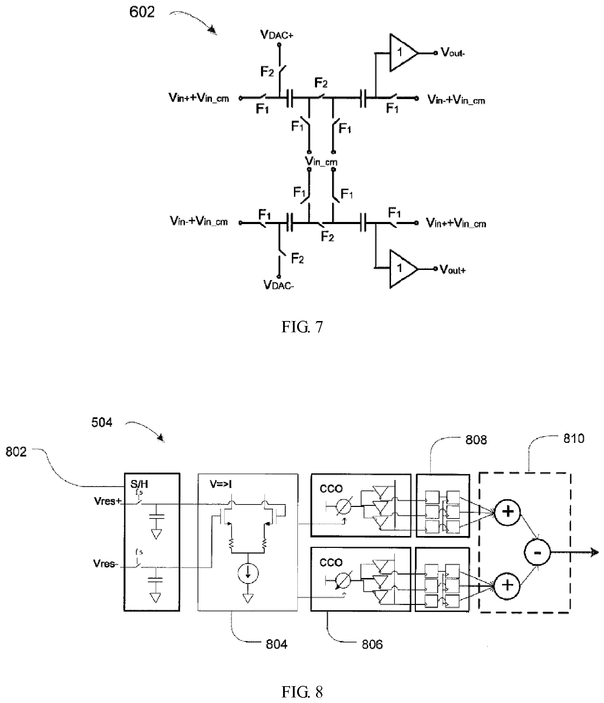

As mentioned above, the voltage controlled oscillator requires a much smaller input voltage level than the conventional voltage mode analog-to-digital converter, so that the residue of the first stage analog-to-digital converter can be converted directly by a voltage-controlled oscillator without a large gain. Therefore, in one embodiment of this disclosure, a voltage multiplying circuit can be used to amplify the residual signals. FIG. 7 shows a circuit diagram of the residual amplifier shown in FIG. 6. In this embodiment, the voltage multiplying circuit 602 is a switched capacitor circuit, which realizes the signal processing through the on-off of different phase switches. In FIG. 7, V_{in_cm} is, for example, the grounding of AC. During the first phase, all the switches labeled as F₁ are closed, and all the switches labeled as F₂ are opened, and Vin+ and Vᵢₙ₋ are stored separately on 4 sampling capacitors. During the second phase, all the switches labeled as F₁ are open, and all the switches labeled as F₂ are closed, the Vₒᵤₜ₋ voltage is changed to (Vᵢₙ₋-Vᵢₙ₊), and the Vₒᵤₜ₊ voltage becomes (Vin+-Vin-), and the final differential output signal is 2* (Vin+-Vin-), realizing the multiplying of the input differential signal. The voltage multiplying circuit shown in FIG. 7 has the advantages of simple structure and low power consumption, and the gain magnified residual signal is suitable for the input of the VCO.

FIG. 8 is a block diagram showing the second stage analog-to-digital converter in FIG. 6. The second stage analog-to-digital converter 504 comprises a sample-and-hold circuit 802, a voltage to current conversion circuit 804, a current-controlled oscillator 806, a bidirectional counter 808, and an addition and subtraction device (displayed in a dashed frame) 810, in which the voltage to the current conversion circuit 804 and the current-controlled oscillator 806 constitute a voltage controlled oscillator described above.

The sample-and-hold circuit 802 receives the first residual analog signal from the residual amplifier 602 and samples the first residual analog signal according to the control of the switching signal fs and keeps the signal on the sampling capacitor to generate a differential sampling voltage signal. The differential sampling voltage signal is then input to the voltage to current conversion circuit 804. The voltage to current conversion circuit 804 is a differential input circuit of the source negative feedback (source degenerated), which comprises two branches coupled to a current source; each of the two branches comprises series-connected NMOS transistors and resistors. The gates of the NMOS transistors are coupled to the sample and hold circuit 802 to receive differential sampling voltage signals. The drains of the NMOS are directly or indirectly coupled to the current output end of the voltage to current converter to provide sampled current signals to the current-controlled oscillator 806.

The current-controlled oscillator (CCO) 806 generates oscillating signals based on the sampling current signals. The oscillation frequency of the oscillating signal is proportional to the input control current so that the frequency difference of the two current regulated oscillators is proportional to the current signal of the differential input, and is also proportional to the voltage signal of the differential input. The bidirectional counter 808 counts according to the oscillating signal. The oscillation frequencies of the two current-controlled oscillators are recorded by two bidirectional counters. The addition and subtraction device 810 then calculates the digital signal based on the count result from the bidirectional counter 808.

More specifically, the addition and subtraction device 810 performs the subtraction of the counts of the voltage signals of the differential input to generate a count result proportional to the frequency difference between the two current-controlled oscillators, which is a second digital signal directly proportional to the input differential voltage. The digital signal is then calibrated by the digital calibration circuit 506, and is combined with the first digital signal to generate a digital signal. The digital signal is then processed by the DSP in the second chip 206 to generate image data for display.

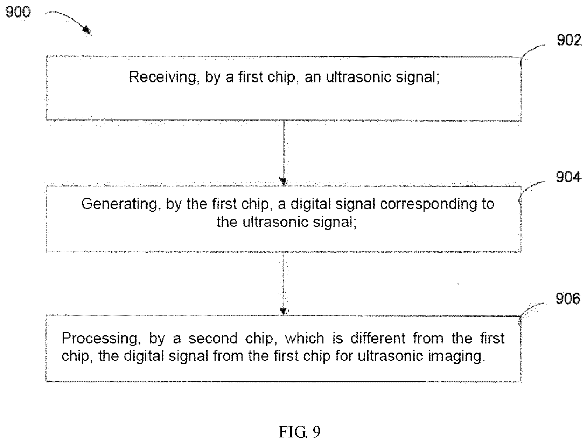

FIG. 9 shows a flow chart of the methods according to some embodiments of the disclosure. The method 900 can be implemented, for example, by equipment in FIG. 2 and FIG. 3. In step 902, the ultrasonic signal is received by using the first chip 202. The first chip 202 comprises terminals connected to external components to receive analog electrical signals corresponding to ultrasonic signals from ultrasonic transducers.

In step 904, the digital signal corresponding to the ultrasonic signal is generated by using the first chip 202. As described above, there are a plurality of channels 300 in the first chip 202, each of which comprises LNA302, VGA 304 and ADC 306 for low noise amplification, variable gain amplification and analog digital conversion for analog electrical signals representing ultrasonic signals, thus generating digital signals representing ultrasonic signals. By redesigning the LNA302 and ADC 306, the power consumption in the ultrasonic signal amplification and conversion can be greatly reduced, so that the method can be applied to the portable ultrasonic equipment.

In step 906, the second chip 206, which is different from the first chip 202, processes the digital signal from the first chip for ultrasonic imaging. In the disclosure, a second chip made of ASIC technology comprises a digital signal processor (DSP). Compared to the traditional ultrasonic detector solutions, where the field programmable gate array (FPGA) chip is used to realize the digital signal processing function of the ultrasonic detection, the second chip made by ASIC technology can obtain more optimized circuit power and area, thus reducing the production cost of the chips. The second chip 206 performs filtering, equalization and noise reduction for the ultrasonic pulse feedback signals amplified and digitized through an analog front end, and sends the processing results to the operating system (OS) of the ultrasonic detector and the user interface (UI) for analysis and judgment.

In general, the disclosure provides a device and method for ultrasound imaging. The device and method are especially suitable for portable ultrasonic imaging devices because of their low power consumption and small volume. Compared with the conventional ultrasonic imaging devices and methods, the disclosure creatively separates the functional imaging components of conventional ultrasonic devices, redesigns and divides them into two separate chips, followed by integrating the components into one package through the 3D encapsulation technology thus optimizing the power and area. In addition, the embodiments of the disclosure can greatly reduce the power consumed by the analog front end by redesigning the circuits in the analog front end chip, especially the low noise amplifier and the analog-to-digital converter, so that the device used for ultrasonic imaging according to the embodiment of the disclosure can be applied to the portable ultrasonic imaging device.

Although the disclosed equipment is described as a separate component, it can be understood that at least some of the parts of these components may be implemented as a whole in some of the embodiments. Although various aspects of the disclosure are shown and described as block diagrams, flowcharts, or some other drawings, it is understood that the frames, devices, systems, techniques, or methods described herein may be implemented in a nonrestrictive manner in the form of hardware, software, firmware, dedicated circuits or logics, general hardware or controllers, computing devices, or a combination thereof.

In addition, although the operations are described in a specific order, this should not be understood as requiring such operations to be executed in the order shown or executed in sequential sequences, or to require all operations to be executed to achieve the desired results. In some cases, multitask or parallel processing can be advantageous. Similarly, although the details of a number of specific implementations are included in the discussion above, these should not be interpreted as any restrictions on the scope of the disclosure, and the description of the characteristics is only for specific implementations. Some features described in some of the separate embodiments can also be performed cooperatively in a single embodiment. Likewise, the various characteristics described in a single embodiment can also be implemented separately in multiple embodiments or implemented in any suitable subcombination.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. A device for ultrasonic imaging, comprising:
a first chip, being configured to receive an ultrasonic signal, the ultrasonic signal being an analog signal, and to generate a digital signal corresponding to the ultrasonic signal; and
a second chip, being configured to process the digital signal transmitted from the first chip for ultrasonic imaging.

2. The device of claim 1, wherein the first chip is stacked on the second chip to form a three-dimensional stacked chip package.

3. The device of claim 1, wherein the second chip comprises a digital signal processor prepared by an application-specific integrated circuit technology.

4. The device of claim 1, wherein the first chip comprises a plurality of signal channels, each of which comprises:
a low noise amplifier configured to generate a first amplification signal by amplifying the ultrasonic signal;
a variable gain amplifier configured to generate a second amplification signal by amplifying the first amplification signal; and
an analog-to-digital converter configured to convert the second amplification signal into the digital signal.

5. The device of claim 4, wherein the low noise amplifier comprises a differential current reuse low noise amplifying circuit; the differential current reuse low noise amplifying circuit comprises:
a first branch circuit comprising a first P type metal oxide semiconductor transistor and a first N type metal oxide semiconductor transistor which are connected in series;
a second branch circuit comprising a second P type metal oxide semiconductor transistor and a second N type metal oxide semiconductor transistor which are connected in series; and
gates of the first and second P type metal oxide semiconductor transistors and the first and second N type metal oxide semiconductor transistors are coupled with an input capacitor to receive a differential input signal; drains of the first and second P type metal oxide semiconductor transistors and the first and second N type metal oxide semiconductor transistors output the first amplification signal.

6. The device of claim 4, wherein the analog-to-digital converter comprises:
a first stage converter configured to generate a converted first digital signal and an amplified first residual analog signal according to the second amplification signal;
a second stage analog-to-digital converter based on a voltage controlled oscillator, which is configured to generate a second digital signal according to the first residual analog signal; and
a digital calibration circuit configured to generate a calibrated digital signal according to the first digital signal and the second digital signal.

7. The device of claim 6, wherein the first stage converter comprises:
a flash analog-to-digital converter configured to generate the first digital signal according to the second amplification signal and a first reference signal;
a digital to analog converter configured to generate a first analog signal according to the first digital signal and the first reference signal;
an addition and subtraction device configured to generate a residual signal according to the second amplification signal and the first analog signal; and
a residual amplifier configured to generate a first residual analog signal according to the residual signal.

8. The device of claim 7, wherein the residual amplifier comprises a voltage multiplier circuit based on a charge pump; the voltage multiplier circuit is configured to:
store paired residual signals in a first stage, and
combine and output the paired residual signals in a second stage to yield the first residual analog signal.

9. The device of claim 6, wherein the second stage analog-to-digital converter comprises:
a sample-and-hold circuit configured to sample and maintain the first residual analog signal to generate a sampling voltage signal;
a voltage to current conversion circuit configured to convert the sampling voltage signal to a sampling current signal;
a current-controlled oscillator configured to generate an oscillating signal based on the sampling current signal;
a bidirectional counter configured to count according to the oscillating signal; and
an addition and subtraction device configured to calculate the second digital signal according to a counting result from the bidirectional counter.

10. A portable ultrasonic testing equipment, comprising:
an ultrasonic transducer configured to generate an ultrasonic signal by detection;
a device for ultrasonic imaging of any one of claims 1-9, which is configured to generate a digital signal for ultrasonic imaging according to the ultrasonic signal; and
a display unit configured to image according to the digital signal.

11. A method of ultrasonic imaging, comprising:
receiving, by a first chip, an ultrasonic signal, the ultrasonic signal being an analog signal;
generating, by the first chip, a digital signal corresponding to the ultrasonic signal; and
processing, by a second chip, which is different from the first chip, the digital signal transmitted from the first chip for ultrasonic imaging.

12. The method of claim 11, wherein the first chip is stacked on the second chip to form a three-dimensional stacked chip package.

13. The method of claim 11, wherein the second chip comprises a digital signal processor prepared by an application-specific integrated circuit technology.

14. The method of claim 11, wherein the first chip comprises a plurality of signal channels, each of which comprises a low noise amplifier, a variable gain amplifier, and an analog-to-digital converter; generating, by the first chip, a digital signal corresponding to the ultrasonic signal comprises:
amplifying, by the low noise amplifier, the ultrasonic signal to generate a first amplification signal;
amplifying, by the variable gain amplifier, the first amplification signal to generate a second amplification signal; and
converting, by the analog-to-digital converter, the second amplification signal into the digital signal.

15. The method of claim 14, wherein the low noise amplifier comprises a differential current reuse low noise amplifying circuit; the differential current reuse low noise amplifying circuit comprises: a first branch circuit comprising a first P type metal oxide semiconductor transistor and a first N type metal oxide semiconductor transistor which are connected in series, and a second branch circuit comprising a second P type metal oxide semiconductor transistor and a second N type metal oxide semiconductor transistor which are connected in series;
generating the first amplification signal comprises:
receiving, by gates of the first and second P type metal oxide semiconductor transistors and the first and second N type metal oxide semiconductor transistors, a differential input signal; and
outputting, by drains of the first and second P type metal oxide semiconductor transistors and the first and second N type metal oxide semiconductor transistors, the first amplification signal.

16. The method of claim 14, wherein the analog-to-digital converter comprises: a first stage converter, a second stage analog-to-digital converter based on a voltage controlled oscillator, and a digital calibration circuit;
converting the second amplification signal into the digital signal comprises:
generating, by the first stage converter, a converted first digital signal and an amplified first residual analog signal according to the second amplification signal;
generating, by the second stage analog-to-digital converter, a second digital signal according to the first residual analog signal; and
generating, by the digital calibration circuit, a calibrated digital signal according to the first digital signal and the second digital signal.

17. The method of claim 16, wherein the first stage converter comprises: a flash analog-to-digital converter, a digital to analog converter, an addition and subtraction device, and a residual amplifier;
generating the first digital signal comprises:
generating, by the flash analog-to-digital converter, the first digital signal according to the second amplification signal and a first reference signal;
generating the first residual analog signal comprises:
generating, by the digital to analog converter, a first analog signal according to the first digital signal and the first reference signal;
generating, by the addition and subtraction device, a residual signal according to the second amplification signal and the first analog signal; and
generating, by the residual amplifier, the first residual analog signal according to the residual signal.

18. The method of claim 17, wherein the residual amplifier comprises a voltage multiplier circuit based on a charge pump;
generating the first residual analog signal comprises:
storing, by the voltage multiplier circuit, paired residual signals in a first stage, and
combining and outputting, by the voltage multiplier circuit, the paired residual signals in a second stage to yield the first residual analog signal.

19. The method of claim 16, wherein the second stage analog-to-digital converter comprises: a sample-and-hold circuit, a voltage to current conversion circuit, a current-controlled oscillator, a bidirectional counter, and an addition and subtraction device;
generating the second digital signal comprises:
sampling and maintaining, by the sample-and-hold circuit, the first residual analog signal to generate a sampling voltage signal;
converting, by the voltage to current conversion circuit, the sampling voltage signal to a sampling current signal;
generating, by the current-controlled oscillator, an oscillating signal based on the sampling current signal;
counting, by the bidirectional counter, according to the oscillating signal; and
calculating, by the addition and subtraction device, the second digital signal according to a counting result from the bidirectional counter.

20. An imaging method of portable ultrasonic testing equipment, the method comprising:
generating an ultrasonic signal according to ultrasonic energy exchange;
generating, by the device for ultrasonic imaging of any one of claims 1-9, a digital signal for ultrasonic imaging according to the ultrasonic signal; and
imaging according to the digital signal.

21. A method of producing ultrasonic imaging device, the method comprising providing a component unit of the device for ultrasonic imaging of any one of claims 1-9.
